Europäisches Patentamt

European Patent Office

Office européen des brevets

(19)

(11) Publication number: 0 321 929
A1

# (12) EUROPEAN PATENT APPLICATION

(21) Application number: 88121312.8

(51) Int. Cl.⁴: A61K 7/42

(22) Date of filing: 20.12.88

The title of the invention has been amended (Guidelines for Examination in the EPO, A-III, 7.3).

(30) Priority: 21.12.87 US 135666

(43) Date of publication of application:
28.06.89 Bulletin 89/26

(84) Designated Contracting States:
BE CH DE FR GB IT LI

(71) Applicant: ESTEE LAUDER INC.
767 Fifth Avenue
New York New York 10153(US)

(72) Inventor: Smith, Walter P.
89 Blackberry Drive
Stamford Connecticut 06903(US)
Inventor: Marenus, Kenneth D.
116 Grove Road
Kings Park New York 11754(US)
Inventor: Pelle, Edward
140 Meyer Avenue
Valley Stream New York 11580(US)

(74) Representative: Vossius & Partner
Siebertstrasse 4 P.O. Box 86 07 67
D-8000 München 86(DE)

(54) Sunscreen composition containing copper 3,5-diisopropyl salicylate.

(57) An improved sunscreen composition containing an effective amount of copper 3'5' diisoprophylsalicylate (CuDIPS), and its use in preparations for protecting skin from damage caused by ultraviolet light are disclosed. When added to commercial sunscreen compositions, CuDIPS dramatically increases SPF values.

EP 0 321 929 A1

## SUNSCREEN COMPOSITION CONTAINING CuDIPS

The present invention relates to an improved sunscreen composition containing an effective amount of copper 3'5' diisopropylsalicylate (CuDIPS), and to a method of protecting skin from damage caused by exposure to ultraviolet light comprising applying an effective amount of the composition to the skin.

It is well documented that human skin is sensitive to sunlight, as well as artificial light, containing radiation of wavelengths between about 290 nanometers (nm) and 400 nm. Ultraviolet radiation of wavelengths between about 290 nm and 320 nm (UV-B region) has long been known to produce damaging effects on the skin including reddening or erythema, edema, blistering and other skin eruptions. Prolonged or chronic exposure to radiation of this wavelength range has been associated with serious skin conditions such as actinic keratoses and carcinomas. In recent years, concern has also been expressed regarding ultraviolet radiation of wavelengths above 320 nm (UV-A region) and the adverse effects of such radiation on human skin.

Currently, the most widely utilized commercial sunscreen agents include para-amino benzoic acid derivatives, oxybenzones, and methoxycinnamates. These sunscreen agents can be formulated into a wide range of compositions having sun protection factor (SPF) values that typically range from 2-30.

SPF is the ratio of the minimal erythemal dose (MED) of protected skin to the MED of unprotected skin.

$$SPF = \frac{MED \text{ of protected Skin}}{MED \text{ Of unprotected Skin}}$$

MED is defined as that amount of UV energy required to induce a slight inflammation of the exposed site. In other words, the effectiveness of a UV blocking product is determined by the amount of extra UV irradiation that is required to achieve the same biological response in a protected site as in an unprotected site.

It is an object of the present invention to provide improved sunscreen compositions that include CuDIPS to provide additional protection to the skin. As we shall discuss below, when CuDIPS is used in combination with sunscreen agents, compositions are obtained which have substantially superior SPF values as compared to compositions that do not contain CuDIPS.

The present invention is directed to an improved sunscreen composition comprising effective amounts of a suncreen agent and CuDIPS in a cosmetically acceptable carrier, and to a method of using the composition to protect the skin from the adverse effects of light containing radiation of wavelengths within the range of 290-400 nm.

The sunscreen agent of the composition of our invention preferably comprises a sunscreen agent selected from the group consisting of para-amino benzoic acid derivatives, oxybenzones, methoxycinnamates, homosalates, octyl salicylates and mixtures thereof. Such sunscreen agents are well-known in the art.

The composition of our invention may be applied to the skin in any suitable form, for example as a lotion, cream or gel. As will be appreciated by persons skilled in the art, the form of the composition will depend on the cosmetically acceptable carrier for the active ingredients of the composition. The composition, of course, may also include one or more fragrances, as well as additional active ingredients for treating the skin.

Tests have shown that the addition of CuDIPS to presently available sunscreens substantially boosts the SPF values of the resulting composition to surprisingly high levels. These tests, which were performed by an independent testing lab, followed the procedure outlined in the "Proposed Monograph for OTC Sunscreen Drug Products" issued by the Food and Drug Administration, August 25, 1978, Federal Register Volume 43, No. 166, pp. 38206-38269.

The sunscreen samples that were provided to the testing lab included a number of commercial Estee Lauder sunscreen products to which we added 0.015% by weight CuDIPS. The independent lab reported to us that the results set forth in Table I were obtained when the various samples submitted were tested for SPF values

TABLE I

| Commercial Product | SPF without added CuDIPS | SPE with added CuPIPS |
|---|---|---|
| Aramis Extra Protection | 15.5 | 19.0 |
| Rx Adv. Sun Protection | 6.4 | 8.1 |
| Estee Lauder Super Sun Block | 20.1 | 22.9 |
| Estee Lauder Golden Tanning Milk | 4.3 | 5.2 |
| Estee Lauder Tanning Milk | 4.0 | 6.5 |

The results set forth in Table I show that the addition of small amounts of CuDIPS to a number of commercial sunscreen products substantially enhances the SPF values of those products. Even though CuDIPS is capable of a nominal amount of UV absorption, that amount of UV absorption is relatively insignificant as compared to the large boosts in SPF values that result when CuDIPS is combined with sunscreen agents.

The results set forth on Table I are particularly surprising because, of a number of free radical scavengers that we have tested in combination with a commercial sunscreen product, only CuDIPS free radical scavenger substantially boosted the SPF value of the resulting composition. In these tests we used Estee Lauder Tanning Milk, which as noted in Table I has an SPF value of 4.0, when no CuDIPS was added to it, and an SPF value of 6.5 when 0.015% by weight CuDIPS was added. However, when the following free radical scavengers in the stated amounts were added to Estee Lauder Tanning Milk, there was no change in the SPF value of the commercial product: Vitamine E (5%); Superoxide Dismutase (5%); Pantethine (2%); Mannitol (5%); and Catalase (5%).

We believe that the compositions of the present invention are also capable of reversing, to at least some extent, damage to the skin that may have already been caused by ultraviolet light.

In preferred embodiments, the composition of the present invention comprises:

(a) from about 2 to about 23 per cent by weight of the composition of a sunscreen agent;

(b) from about 0.01 to about 0.02 percent by weight of the composition of CuDIPS; and

(c) a cosmetically acceptable carrier.

The ingredients used in the composition of this invention should be of a quality or purity (such as U.S.P. or N.F.) suitable for cosmetic use and should be compatible when used together in a particular composition.

A number of commercially available sunscreen agents may be used in the present invention. Particularly preferred sunscreen agents are paraamino benzoic acid derivatives, oxybenzones, methoxycinnamates, homosalates, and octyl salicylates.

CuDIPS suitable for use in our composition is available from Laboratoires Serobiologiques, Inc.

The cosmetically suitable carrier is selected to obtain the desired form of the final composition, e.g., a gel, a lotion or a cream. Particularly preferred carriers include non-ionic emulsions and triethanolamine/stearic acid anionic emulsions.

The preferred sunscreen composition emulsions of this invention may be made by mixing techniques known in the art. For example, the emulsions of the present invention may be made by the following general procedure:

a) Adding the components of the oil phase of the emulsion to each other and then mixing at a temperature of 80°C;

(b) Adding the components of the water phase to each other, with mixing at a temperature of 78°C.

(c) Adding the oil phase to the water phase, with mixing, to homogenize the mixture.

(d) After homogenization, adding triethanolamine and deionized water with mixing, at 70°C.

(e) Cooling the mixture to 40°C, with mixing, (and adding CuDips to the mixture at 40°C.

(f) Cooling the mixture to 30°C, at which time any additive ingredients (e.g., maskants or fragrances) may be added, continue mixing.

(g) Cooling to room temperature, with mixing.

The composition of the present invention may be applied to the skin as often as is necessary to provide adequate protection. Factors such as the length of time spent outside or in water, the condition of the skin, skin type, etc., will determine how frequently the compositions of the present invention are most desirably applied to the skin.

The following non-limiting Examples illustrate the present invention, with all percentage stated based on the total weight of the composition:

· EXAMPLE 1

NON-IONIC EMULSION SYSTEM

A sunscreen composition was prepared from the following ingredients in the specified amounts

| Oil Phase Ingredients | % By Weight |
|---|---|
| Cinnamate Sunscreen | 7.5 |
| Oxybenzone Sunscreen | 6.0 |
| Octylsalicylate Sunscreen | 5.0 |
| Bentone Gel Thickener | 6.0 |
| Rice Oil Emolient | 4.0 |
| Stearic Acid Emolient | 3.0 |
| Amphisol Emulsifer | 2.5 |
| Cetiol Emolient | 2.5 |
| Ganex Resin | 2.0 |
| Shea Butter Emolient | 0.2 |
| Propyl Paraben Preservation | 0.15 |
| Butyl Paraben Preservation | 0.10 |
| CuDips | 0.015 |
| Water Phase Ingredients | |
| Carbopol Thickener | 10.0 |
| Sorbitol Humectant | 5.0 |
| Methyl Paraben Preservative | 0.3 |
| Germall 115 Preservative | 0.3 |
| Emulsifer Triethanolamine | 0.2 |
| Deionized Water | 45.235 |

The above ingredients were mixed and processed. as described in steps (a)-(g) above to obtain a sunscreen composition.

EXAMPLE 2

TRIETHANOLAMINE/STEARIC ACIDANIONIC EMULSION

A sunscreen composition was prepared from the ingredients stated below, in the amounts stated.

| Oil Phase Ingredients | % By Weight |
|---|---|
| Parsol MCX (Methoxycinnamate) Sunscreen | 4.0 |
| PURECO 76 Emolient/oil | 3.5 |
| Robane Emolient/oil | 3.5 |
| Glyceryl Monostearate Wax | 3.0 |
| Lexol GT-865 Emolient/oil | 2.5 |
| Stearic Acid 132 Emulsifier | 2.2 |
| Shea Butter Emolient | 0.25 |
| Almond Oil Emolient | 0.20 |
| Butyl Paraben Preservative | 0.10 |
| Propyl Paraben Preservative | 0.10 |
| CuDips | 0.015 |
| Water Phase Ingredient | |
| Carpopol Thickener | 9.0 |
| Propylene Glycol Humectant | 6.0 |
| Triethanolamine Emulsifer | 1.15 |
| Germall 115 Preservative | 0.3 |
| Methyl Paraben Preservative | 0.3 |
| Propyl Paraben Preservative | 0.05 |
| Deionized Water | 63.835 |

The above ingredients were mixed and processed, as described in steps (a)-(g) above, to obtain a sunscreen composition.

## Claims

1. A composition for protecting skin from damage caused by exposure to ultraviolet light, said composition comprising a sunscreen agent and copper $3'5'$ diisopropylsalicylate in a cosmetically acceptable carrier.

2. The composition of claim 1, wherein the composition comprises from about 2 to about 23 percent by weight of the sunscreen agent and from about 0.01 to about 0.02 percent by weight of copper $3'5'$ diisopropyllicylate.

3. The composition of claim 1, wherein the sunscreen agent is selected from the group consisting of para-amino benzoic acid derivatives, oxybenzones, methoxycinnamates, homosalates, octyl salicylates and mixtures thereof.

4. The composition of claim 1 in the form of a non-ionic emulsion.

5. The composition of claim 1 in the form of a triethanolamine/stearic acid anionic emulsion.

6. The use of an effective amount of copper $3'5'$ diisopropylsalicylate for the preparation of compositions for protecting skin from damage caused by exposure to ultraviolet light.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | EP-A-0 293 579 (L'OREAL) <br> * Whole document * <br> ----- | 1-6 | A 61 K 7/42 |

TECHNICAL FIELDS
SEARCHED (Int. Cl.4)

A 61 K

The present search report has been drawn up for all claims

| Place of search | Date of completion of the search | Examiner |
|---|---|---|
| THE HAGUE | 03-02-1989 | FISCHER J.P. |

CATEGORY OF CITED DOCUMENTS

X : particularly relevant if taken alone
Y : particularly relevant if combined with another
　document of the same category
A : technological background
O : non-written disclosure
P : intermediate document

T : theory or principle underlying the invention
E : earlier patent document, but published on, or
　after the filing date
D : document cited in the application
L : document cited for other reasons

& : member of the same patent family, corresponding
　document

EPO FORM 1503 03.82 (P0401)